# EUROPEAN PATENT APPLICATION

(11) **EP 2 072 069 A1**
(43) Date of publication of application: **24.06.2009**
(21) Application number: 07025024.6
(22) Date of filing: 21.12.2007
(51) Int. Cl.: A61L 31/14, A61F 2/00, A61L 27/50, A61L 29/14, A61M 25/00, B05D 3/06, C08J 7/12, C08J 9/00

(54) **Fluorinated polymers in medical devices**

(71) Applicant: Abbott Laboratories Vascular Enterprises Limited, Terrace, Dublin 2 (IE)
(72) Inventor: Marx, Volker, 72379 Hechingen (DE)
(74) Representative: Peters, Hajo

(57) **Abstract**

The present invention refers to medical devices comprising fluorinated polymers, especially to tubings, used for constructing catheters like balloon catheters, delivery catheters or guide catheters.

## Description

### Field of the invention

The present invention refers to medical devices comprising fluorinated polymers, especially to tubings, used for constructing catheters like balloon catheters, guide catheters or delivery catheters

### Background of the invention

Angioplasty is an efficient and successful method of opening stenosis in the vascular system. In a popular form of angioplasty, a balloon catheter is advanced through the vascular system until the balloon, which is carried at the distal end of a catheter shaft, and which may carry an expandable stent, is positioned across the stenosis or damaged vessel. By inflating the balloon pressure is applied to the obstruction, which is moved by pressing it against the inner wall of the vessel, whereby the vessel is opened for improved flow. Due to the expansion of the balloon, the stent, which - if used - is situated on the balloon, is also expanded for aiding in repairing the vessel wall and hindering obstruction. As a last step the stent is then released by deflating the balloon reducing its circumference until refolding of the balloon occurs followed by removal of the balloon and catheter from the vessel along a guide wire.

There are various types of balloon catheters. One type is fed over a guide wire (i.e., "over-the-wire" catheters) and another type serves as its own guide wire ("fixed - wire" catheters). There have been developments of variations of these two basic types: the so-called "rapid exchange" type, "innerless" catheters, and others.

In another popular form of angioplastic selfexpanding stents are delivered to the target site in a vessel via a self-expanding stent delivery catheter. Usually the stent is covered by a sheath to keep it in the contracted configuration on the catheter until it reaches the target side. The sheath is then retracted in order to release the stent and the catheter is subsequently removed from the vessel.

If a catheter, like a balloon catheter, is used in percutaneous transluminal angioplasty (PTA) or percutaneous transluminal coronary angioplasty (PTCA), it is typically advanced through a guide catheter to a preselected vessel location, such as the aorta, for example. Using fluoroscopy, the surgeon advances the catheter until the balloon is located across the stenosis or obstruction. This may involve the use of a guide wire over which the catheter is moved or alternatively the catheter may act as its own guide wire. Besides the fact that a smooth advancement in the vessels is in itself very advantageously, a possible origin of trouble arriving from this advancement of the catheter is the friction between the wire and the inner lumen of the catheter when guiding the catheter along the wire. As the movement in the vessels is highly difficult and a task requiring great handling skills any resistance in addition to those unavoidable due to the nature of the vessels being penetrated is strictly unwanted. Accordingly besides smoothness of the catheter (tip) itself a smooth movement of the wire inside the catheter avoiding as much friction as possible is highly desired.

One solution used for reducing friction on the outside of a catheter involves the application of silicone on the surface/s especially by use of silicone oil, but with the high disadvantage that the silicone oil is smeared over the catheter and lost. Another solution often used is the hydrophilic coating, but this process often needs additional difficult steps, including curing under UV radiation.

Polyamides or polyamide elastomers have been used in the polymer industry for a long time and - due to their enormous range of possible applications - are found in many branches of industrial products. Recently in the area of medicinal devices good use has been made of these materials especially in devices/implants like the balloons on a balloon catheter. The most popular polyamides used include different sorts of Nylons or Copolymers such as PEBA. Even though these materials have certainly been used successfully, due to the strains put on the materials and the necessity to improve their characteristics in the light of growing experience coming from increasing numbers of treated patients, there clearly is a need for improved materials allowing for an effective treatment of the patient minimizing risks, preferably with an economical production process.

### Summary of the invention

It is an object of the current invention to provide new medical devices using polymers showing the attributes wanted in their specific area of use. As the special focus of this invention is on the search for new materials to be used wherever low-friction characteristics and a high slideability is required. For example in the inner member, especially the guide wire lumen of medical devices like balloon catheters, used e.g. in PTA (Percutaneous transluminal angioplasty) or PTCA (Percutaneous transluminal coronary angioplasty) a material showing the lowest amount of friction in contact with the guide wire is needed and also advantages in the production steps.

The invention thus refers to a medical device comprising a fluorinated polymer, especially to a catheter like a catheter with a balloon or a delivery catheter for delivery of medical devices or a guide catheter like those used in PTA/ PTCA/ angioplastic applications. Desirably at least one surface of the medical device is consisting of a fluorinated polymer. Medical devices, especially catheters with their outer surface or any inner surface formed by this fluorinated polymer do show a greatly increased sliding property which in regards to for example the friction between guide wire and inner surface of the guide wire lumen or friction between the guiding catheter and the catheter or the self expanding stent and the covering sheath of the delivery catheter is improved over those currently used in the state of the art. Furthermore fluorination improves protection against microorganisms and bacteria and additionally transfers its attributes for a long duration of time, maybe over years. In addition the material shows a positive effect on a balloon mounted on a catheter for balloon angioplastic applications as such a balloon shows improved surface characteristics such as smoothness or as a diffusion barrier is introduced improving the balloon on inflation.

The invention further rests in a method for improving the sliding properties of a surface in a catheter by conveying gaseous fluorine to the surface of the catheter whose surface (S) before application of the fluorine gas is consisting of non-fluorinated polymer.

The invention further rests in a method of producing a medical device or tubing used for forming a medical device with at least one surface (S) consisting of a fluorinated polymer, wherein this surface is treated with gaseous fluorine. Thereby, the medical device itself or being produced is a catheter, like a balloon catheter for balloon dilatation or a catheter for stent delivery or a guide catheter.

The invention further rests in the use of fluorinated polymers in the manufacture of a medical device, desirably selected from stents, stent grafts, grafts, graft connectors or catheters, but most desirably from a catheter.

The invention also further rests in the use of a medical device according to the invention for the treatment of a disease, especially a cardiovascular disease, especially a stenosis, especially through minimal invasive surgery like PTCA.

In a further embodiment the invention is directed to a method of treatment of a disease, like a cardiovascular disease, especially a stenosis, using in a patient, being a mammal, especially a human, in need thereof a medical device according to the invention, desirably in minimal invasive surgery like PTCA.

As a last aspect the invention furthermore resides in medical devices comprising fluorinated metal. As metals can also be fluorinated, so-called hypotubes often used in catheters of the "rapid-exchange" type can be improved by surface fluorination or even whole catheter assemblies like a balloon catheter with a metal stent mounted on the balloon or a delivery catheter bearing a self-expandable stent or other medical device can be treated with gaseous fluorine and thus surface properties can be improved avoiding common known problems of the application of silicone or the hydrophilic coating.

Furthermore, the sliding properties of guidewires usually made from metal can also be improved by surface fluorination.

### Detailed Description of the invention

The use of catheters as well as stents, balloons and other medical devices etc. in minimal invasive surgery, especially in the cardiovascular field, has in the last years shown a high growth. As a consequence the need for useful materials fulfilling highly specialized needs in the field of different medicinal devices has clearly risen in a technical area, which traditionally is more governed by bulk products. Especially in the field of vascular catheters being typically advanced through a guide catheter during PTCA, a material being easily producible as well as offering less friction, either in the outside of the catheter or between the wire and the inner lumen of the catheter when guiding the catheter along the wire is very desired. In addition the problems involved with the common methods for reducing friction on the outside of a catheter are not optimized (smearing of silicone and additional steps, e.g. UV-curing) and the corresponding production steps need improving.

Kinds of Polymers commonly used for medical devices include for example
a) Nylon: Nylon, coming in different sorts, especially Nylon-12, are not very flexible and/or have a relatively high water absorption. Especially the lack of flexibility is often considered as a drawback in medical devices using Nylon.
b) PEBA: PEBA (e.g. PEBAX^{®}) are often too flexible and/or have a relatively high water absorption In addition PEBA lacks stability, especially in regards to the thermo-oxidation and/or sometimes also lack dimensional stability.
c) Blend of a) and b): The need for a compromise between the higher rigidity of Nylon and higher flexibility of PEBA has already resulted in blends being used. A disadvantage of blends is that the phases tend to show phase separation that leads to unstable morphology.

None of these does show an optimal behavior, especially if used for catheters or the inner member of catheters, so that still there is a high need for appropriate material for medical devices, especially for catheters.

The invention thus refers to a medical device comprising a fluorinated polymer. Thereby it is preferred if the medical device according to the invention is selected from implanted,implantable or minimal invasive medical devices, preferably stents, stent grafts, grafts, graft connectors, medical balloons or catheters, more preferably the medical device is a catheter, most preferably is a catheter being used with a guide wire or a catheter being used with a guide wire and carrying a balloon, optionally with a stent, or delivery catheter comprising a retractable sheath or a guide catheter. Most desirably at least one surface of the medical device is consisting of a fluorinated polymer, preferably either the outer surface of the catheter, or an inner surface of the catheter, or both surfaces is/are consisting of a fluorinated polymer, most preferably the inner surface of the guide wire lumen of the catheter using a guide wire is/are consisting of a fluorinated polymer, or the inner surface of a guide catheter is/are consisting of a fluorinated polymer or the inner surface of the retractable sheath of a delivery catheter is/are consisting of a fluorinated polymer.

Medical devices, especially catheters with any of their surfaces formed by this fluorinated polymer do show a greatly increased sliding property over those currently used in the state of the art. For example catheters with their outer surface or their inner member/guide wire lumen formed by this fluorinated polymer do show a greatly increased sliding property which in regards to the friction between guide wire and guide wire lumen of the catheter is improved over those currently used in the state of the art. Furthermore fluorination improves protection against microorganisms and bacteria and additionally transfers its attributes for a long duration of time, maybe over years. In addition the material shows a positive effect on balloons. The fluorination also provides production advantages not showing the problems involved with the common methods for reducing friction on the outside of a catheter like the smearing of silicone or technically problematic production steps like UV-curing.

Fluorine, a gaseous element, is the most reactive chemical element and is reacting with many polymeric/TPE (thermoplastic elastomer) surfaces, like those of coployester TPEs, olefinic TPEs, styrenic TPEs, elastomeric alloy TPEs, polyurethane TPEs or polyamides. A fluorinated layer is formed on the surface as usually hydrogen is replaced by fluorine. Thus, non-polar surfaces are converted into polar surfaces with the effect of reducing the surface energy and the wettability.

The rest of the polymeric material is not subject to any changes and also the surface is not converted beyond the effect described.

In general there are many possibilities to introduce a direct fluorination through gaseous fluorine. These methods are described in detail by Kharitonov J. of Fluorine Chemistry 103 (2000) 123-127, included here by reference. The ordinary direct fluorination, the method employed here, according to that reference is done by treatment with fluorine or fluorine-inert gas mixtures (nitrogen, helium etc.). The mixture with fluorine inert gas normally does allow a more precise and reliable reaction and control of the fluorination process, leading to a slower and less aggressive reaction with the polymeric surface. In addition in above reference modifications of this procedure like fluorination under static conditions, fluorination in the stream or fluorination during fabrication (using the high temperature of the blow molding process) are specifically described. All of these modifications are suitable for the medical devices according to the invention and the direct fluorination applied in this invention as described by this reference. Furthermore a slight increase of fluorine pressure during the process allows a treatment regardless of the complexity of the treated surface as any small crevice, gap or orifice is reached by the gas. In this regard and also in regards to the use of a fluorine scavenger, like NaF, "catching" potentially destructive HF, reference is made to US 5,214,102, included here by reference. Further articles of interest, also included here by reference are 1) Kharitonov et al. (Surface Coating International Part B: Coatings Transactions, 88, B3; (2005), 157-230) and 2) Kharitonov et al. (J. of Fluorine Chemistry 126 (2005) 251-263).

"Fluorinated Polymer" according to this invention is being defined as a polymer which is obtained by treatment of its surface by gaseous fluorine which is elemental fluorine - in literature (see above) called direct fluorination. Before treatment the polymer is by definition non-fluorinated (not yet fluorinated).

"Stent" means an elongate implant with a hollow interior and at least two orifices and usually a circular or elliptical, but also any other, cross section, preferably with a perforated, lattice-like structure that is implanted into vessels, in particular blood vessels, to restore and maintain the vessels patent and functional.

"Graft" means an elongate implant with a hollow interior and with at least two orifices and usually circular or elliptical, but also any other, a cross section and with at least one closed polymer surface which is homogeneous or, optionally, woven from various strands. The surface preferably is impermeable to corpuscular constituents of blood and/or for water, so that the implant serves as a vascular prosthesis and is usually employed for damaged vessels or in place of vessels.

"Stent graft" means a connection between a stent and a graft. A stent graft preferably comprises a vascular prosthesis reinforced with a stent (both as defined above), wherein a polymer layer is homogeneous or, optionally, woven, knitted plaited etc. from various strands and is either impermeable for corpuscular constituents of blood and/or for water or can also be permeable. More preferably, the stent has on at least 20% of its surface a perforated (lattice-like), preferably metallic, outer layer and at least one closed polymer layer that is located inside or outside the stent outer layer. The closed polymer layer may be homogeneous or, optionally, woven from various strands, and is impermeable for corpuscular constituents of blood and/or for water. Optionally, where the closed polymer layer is disposed inside the metallic outer layer, a further perforated (lattice-like), preferably metallic, inner layer may be located inside the polymer layer.

"Graft connector" means an implant that connects at least two hollow organs, vessels or grafts, consists of the materials defined for grafts or stent grafts and/or has the structure defined for the latter. Preferably, a graft connector has at least two, three or four, orifices, arranged, for example, as an asymmetric "T" shape.

"Catheter" means a tubular instrument intended for introduction into hollow organs. More preferably, a catheter may be designed for use in guiding other catheters, or for angiography, ultrasound imaging, or - especially - balloon catheters for dilatation or stent delivery. This includes also a "Catheter pump" meaning a catheter provided on its tip with a propeller able to assist the pumping of the myocardium.

In a preferred embodiment of the medical device according to the invention in the medical device according to the invention the fluorinated polymer is a polyamide or a Block-Copolymer or a mixture thereof, preferably is a polyamide or a PEBA or a mixture thereof, more preferably is a polyamide, especially the polyamide is Nylon, preferably is Nylon 12 or Nylon 6, most preferably is Nylon 12.

In another preferred embodiment of the medical device according to the invention the fluorinated polymer is obtainable by a process characterized in that a non-fluorinated polymer is exposed to gaseous fluorine, preferably characterized in that a non-fluorinated polymer being either in form of a tubing or already forming at least one surface of a medical device is exposed to gaseous fluorine.
If only a restricted area of a surface is intended to be fluorinated, the areas which shall not be subjected to treatment with gaseous fluorine can be covered by any material gaseous fluorine cannot penetrate like e.g. Teflon or any other polymer thick enough to hinder the gaseous fluorine to permeate. In this embodiment any desired form or pattern of fluorinated area on the surface of the tubing, the medical device or part thereof can be achieved, e.g. longitudinal stripes or a spiral pattern along a tube can be performed or merely the tip portion of a catheter can be treated.

In another highly preferred embodiment of the medical device according to the invention the medical device is either
a) a catheter being used with a guide wire, or
b) a catheter being used with a guide wire and carrying a balloon, or
c) a catheter with a retractable sheath, or
d) a guiding catheter, or
e) a tubing used for forming a), b), c), or d)
with a), b), c), d) and e) comprising a hollow space whose inner surface (S) - before introduction of the fluorine gas - is formed by a non-fluorinated polymer, connecting at least two separate openings. This medical device is obtainable by a process wherein gaseous fluorine is conveyed through the hollow space from one opening to the other. Being a very preferred embodiment this product obtainable through this process is relatively easily produced as the hollow space, preferably the guide wire lumen in a catheter, is a closed lumen and thus specifically the improved sliding abilities are specifically transferred to a clearly defined part of the medical device.

Another aspect of the invention relates to a method for improving the sliding properties of a guide wire in a catheter by conveying gaseous fluorine through the guide wire lumen of the catheter whose inner surface (S) before introduction of the fluorine gas is consisting of non-fluorinated polymer.

A further aspect of the invention refers to a method of producing a medical device or tubing used for forming a medical devices with at least one surface (S) consisting of a fluorinated polymer, characterized in that the surface (S) consisting - before introduction of the fluorine gas - of a non-fluorinated polymer is treated with gaseous fluorine. Desirably the tubing is used in a catheter, preferably in a catheter being used with a guide wire, preferably is forming the inner member forming the guide wire lumen of a catheter being used with a guide wire. Also desirably the medical device is a catheter, like a balloon catheter optionally with a stent, preferably is a catheter being used with a guide wire, preferably the medical device is a catheter being used with a guide wire with the surface forming the guide wire lumen consisting of a fluorinated polymer.
Also desirably the medical device is a guide catheter.
Also desirably the medical device is a delivery catheter for delivery of implantable devices like a filter or a self-expanding stent having a retractable sheath covering the implantable device prior to delivery.

In general as described above in this process including a direct fluorination of an - up to that point not fluorinated - surface of a polymer, there are many possible variants to the process step. Thus, gaseous fluorine or fluorine-inert gas mixtures (of nitrogen, helium etc.) can be used as well as fluorine scavenger, like NaF, and in a further variant the fluorine pressure might be increased during the process. Furthermore, modifications of this procedure like fluorination under static conditions, fluorination in the stream or fluorination during fabrication (using the high temperature of the blow molding process) are all suitable. These reactions are described in more detail in 1) Kharitonov et al. (Surface Coating International Part B: Coatings Transactions, 88, B3; (2005), 157-230) and 2) Kharitonov et al. (J. of Fluorine Chemistry 126 (2005) 251-263); 3) Kharitonov (J. of Fluorine Chemistry 103 (2000) 123-127); 4) US 5,214,102. All of these are included here by reference

Another important aspect of the invention refers to the use of fluorinated polymers in the manufacture of a medical device. Desirably the medical device is selected from implanted, implantable or minimal invasive medical devices, preferably stents, stent grafts, grafts, graft connectors, medical balloons or catheters, more preferably the medical device is a catheter, most preferably is a catheter being used with a guide wire or a catheter being used with a guide wire and carrying a balloon, optionally with a stent, or delivery catheter comprising a retractable sheath or a guide catheter. Most desirably at least one surface of the medical device is consisting of a fluorinated polymer, preferably either the outer surface of the catheter, or an inner surface of the catheter, or both surfaces is/are consisting of a fluorinated polymer, most preferably the inner surface of the guide wire lumen of the catheter using a guide wire is/are consisting of a fluorinated polymer, or the inner surface of a guide catheter is/are consisting of a fluorinated polymer or the inner surface of the retractable sheath of a delivery catheter is/are consisting of a fluorinated polymer.

As a separate aspect the invention furthermore resides in metal-carrying medical devices comprising a fluorinated metal. As metals can also be fluorinated, thus also in e.g. balloon catheters with a metal stent crimped on top or in a catheter with a self-expanding stent the sliding properties can be improved with the problems of the application of silicone or the hydrophilic coating. By coating the metal a very thin layer of a micro-molar thickness of non-reactive material is formed. Desirably the metal-carrying medical device is selected from implanted, implantable or minimal invasive metal-carrying medical devices, preferably stents, stent grafts, grafts, graft connectors, guide wires, or catheters, more preferably is a catheter most preferably is a balloon catheter carrying a stent.

A further aspect of the invention relates to the use of a medical device or metal-carrying medical device according to the invention, for the treatment of a disease, especially a cardiovascular disease, especially a stenosis.

Another aspect and embodiment of the current invention is directed to a method of treatment of a disease, like a cardiovascular disease, especially a stenosis, using in a patient, being a mammal, especially a human, in need thereof a medical device or a metal-carrying medical device according to the invention, desirably in minimal invasive surgery like PTCA.

The examples in the following section are merely illustrative and the invention cannot be considered in any way as being restricted to these applications.

### Examples:

### Example 1: Complete fluorination of a stent carrying balloon catheter

A balloon catheter with a catheter shaft of Nylon 12 and a balloon of PEBAX^{®} with a metal stent crimped on top is introduced into a reaction vessel, in which NaF is present as HF-scavenger.
In a first step the reaction vessel and the medical device are purged by a flow of 100-200 SCCM of nitrogen for up to 1 h. Following that fluorine gas and nitrogen gas are simultaneously introduced into the vessel with a flow rate of between 40 and 45 SCCM for both gases. The reaction time is 12 hours and the reaction is performed at ambient temperature. The fluorine pressure is approximately 40 PSIA. Following that the reaction vessel and the medical device is purged by a flow of 100-200 SCCM of nitrogen for 0.5h and 1 h.

The medical device is removed from the reaction vessel and ready to use, showing over the whole of a superior gliding behaviour without the disadvantages of the application of silicone or hydrophilic coating. Also the metal of the stent is showing a layer of non-reactive fluorine derivatives.

### Example 2: Complete fluorination of a balloon catheter

A balloon catheter with a catheter shaft of Nylon and a balloon of PEBAX^{®} is during the automatic production step introduced into a production area with a gas tight reaction vessel.

In a first step the reaction vessel and the medical device are purged by a flow of 100-200 SCCM of nitrogen for up to 1 h. Following that fluorine gas and nitrogen gas are simultaneously introduced into the vessel with a flow rate of between 35 and 45 SCCM for both gases. The reaction time is 8 hours and the reaction is performed at ambient temperature. Following that the reaction vessel and the medical device is purged by a flow of 100-200 SCCM of nitrogen for 0.5h and 1 h.

Following the further production steps the medical device is moved forward from the reaction vessel.

### Example 3: Complete fluorination of a catheter

A catheter with a polymer surface of Nylon without any balloon or stent is introduced into a reaction vessel, in which NaF is present as HF-scavenger.

In a first step the reaction vessel and the medical device are purged by a flow of 100-200 SCCM of nitrogen for up to 1 h. Following that fluorine gas and nitrogen gas are simultaneously introduced into the vessel with a flow rate of between 25 and 35 SCCM for both gases. The reaction time is 4 hours and the reaction is performed at ambient temperature. The fluorine pressure is 30 PSIA. Following that the reaction vessel and the medical device is purged by a flow of 100-200 SCCM of nitrogen for 0.5h and 1 h.

The medical device is removed from the reaction vessel and ready to use, showing over the whole of a superior gliding behaviour without the disadvantages of the application of silicone or hydrophilic coating.

### Example 4: Fluorination of a catheter

A catheter for use with a guide wire is connected through the guide wire lumen formed by a surface of a non-fluorinated polymer, a Nylon 12, to a source of nitrogen and fluorine gas being connected on both ends of the guide wire lumen thus allowing the gas to be lead back in a closed circle.
In a first step pure nitrogen is introduced into the guide wire lumen to purge the surface for 30 min. This is followed by a mixture of 50% fluorine gas and 50 % nitrogen gas with a flow rate of SCCM for both gases. The reaction time is 6 hours and the reaction is performed at ambient temperature. Following that the guide wire lumen is purged again by a flow of nitrogen for 45 min.

### Example 5: Fluorination of a tubing usable for forming the inner member of a catheter

A tubing consisting of Nylon 12 is connected through the its lumen to a source of nitrogen and fluorine gas being connected on both ends thus forming a closed circle, allowing the gas to be lead back.
In a first step pure helium is introduced into the tubing lumen to purge the surface for 45 min. This is followed by a mixture of 45% fluorine gas and 55 % helium gas with a flow rate of 35 SCCM for both gases. The reaction time is 8 hours and the reaction is performed at ambient temperature. The fluorine pressure is 35 PSIA. Following that the tubing lumen is purged again by a flow of helium for 45 min. The tubing is than laser welded to form a catheter.

## Claims

1. Medical device comprising a fluorinated polymer.

2. Medical device according to claim 1, wherein the medical device is selected from implanted, implantable or minimal invasive medical devices, preferably stents, stent grafts, grafts, graft connectors, medical balloons or catheters, more preferably the medical device is a catheter, most preferably is a catheter being used with a guide wire or a catheter being used with a guide wire and carrying a balloon, optionally with a stent, or a delivery catheter comprising a retractable sheath or a guide catheter.

3. Medical device according to any of claims 1 or 2, wherein at least one surface of the medical device is consisting of a fluorinated polymer.

4. Medical device according to claim 3, wherein
either
the outer surface of the catheter, or
the inner surface of the guide wire lumen of the catheter using a guide wire, or
both surfaces is/are consisting of a fluorinated polymer,
preferably the surface of the guide wire lumen of the catheter using a guide wire is/are consisting of a fluorinated polymer.

5. Medical Device according to claim 3 wherein at least the inner surface of the retractable sheath of a delivery catheter is consisting of a fluorinated polymer.

6. Medical device according to any of claims 1 to 5, wherein the fluorinated polymer is a polyamide or a Block-Copolymer or a mixture thereof or a multilayer thereof, preferably is a polyamide or a PEBA or a mixture thereof, most preferably is a polyamide.

7. Medical device according to claim 6, wherein the polyamide is Nylon, preferably is Nylon 12 or Nylon 6, most preferably is Nylon 12.

8. Medical device according to any of claims 1 to 7, wherein the fluorinated polymer is obtainable by a process **characterized in that** a non-fluorinated polymer is exposed to gaseous fluorine, preferably **characterized in that** a non-fluorinated polymer being either in form of a tubing or already forming at least one surface of a medical device is exposed to gaseous fluorine.

9. Medical device according to any of claims 1 to 8, being either
a) a catheter being used with a guide wire, or
b) a catheter being used with a guide wire and carrying a balloon, or
c) a catheter with a retractable sheath, or
d) a guiding catheter, or
e) a tubing used for forming a), b), c), or d)
with a), b), c), d) and e)comprising a hollow space whose inner surface (S) before introduction of the fluorine gas is formed by a non-fluorinated polymer, connecting at least two separate openings,
obtainable by a process wherein gaseous fluorine is conveyed through the hollow space from one opening to the other.

10. A method for improving the sliding properties of a guide wire in a catheter by conveying gaseous fluorine through the guide wire lumen of the catheter whose inner surface (S) before introduction of the fluorine gas is consisting of non-fluorinated polymer.

11. A method for producing a medical device or tubing used for forming a medical devices with at least one surface (S) consisting of a fluorinated polymer, **characterized in that** the surface (S) consisting before introduction of the fluorine gas of a non-fluorinated polymer is treated with gaseous fluorine.

12. A method according to claim 11, wherein the medical device is a catheter, like a guide catheter or a balloon catheter optionally with a stent or a delivery catheter, preferably is a catheter being used with a guide wire, preferably the medical device is a catheter being used with a guide wire with the inner surface forming the guide wire lumen consisting of a fluorinated polymer.

13. A method according to claim 12, wherein the medical device is a delivery catheter comprising a retractable sheath, preferably the medical device is a delivery catheter comprising a retractable sheath with the inner and/or outer surface forming the retractable sheath consisting of a fluorinated polymer.

14. Use of fluorinated polymers in the manufacture of a medical device.

15. Use according to claim 14, wherein the medical device is selected from implanted, implantable or minimal invasive medical devices, preferably stents, stent grafts, grafts, graft connectors, medical balloons or catheters, more preferably the medical device is a catheter, most preferably is a catheter being used with a guide wire or a catheter being used with a guide wire and carrying a balloon, optionally with a stent, or a delivery catheter comprising a retractable sheath, most preferably is a catheter using a guide wire with the inner surface forming the guide wire lumen consisting of the fluorinated polymer, or is a delivery catheter comprising a retractable sheath with the inner surface of the retraction sheath consisting of the fluorinated polymer.

16. Metal-carrying medical device comprising a fluorinated metal.

17. Metal-carrying medical device according to claim 16, wherein the medical device is selected from implanted, implantable or minimal invasive metal-carrying medical devices, preferably stents, stent grafts, grafts, graft connectors, or catheters, more preferably is a catheter most preferably is a balloon catheter carrying a stent.

18. Use of a medical device according to any of claims 1 to 9 or 16 to 17, for the treatment of a disease, especially a cardiovascular disease, especially a stenosis.
